(19) Europäisches Patentamt
European Patent Office
Office européen des brevets

(11) Publication number: **0 417 803 A1**

(12) **EUROPEAN PATENT APPLICATION**

(21) Application number: **90117694.1**

(51) Int. Cl.[5]: **C07K 5/02,** A61K 37/02

(22) Date of filing: **13.09.90**

The title of the invention has been amended (Guidelines for Examination in the EPO, A-III, 7.3).

(30) Priority: **14.09.89 DE 3930739**

(43) Date of publication of application: **20.03.91 Bulletin 91/12**

(84) Designated Contracting States: **AT BE CH DE DK ES FR GB GR IT LI LU NL SE**

(71) Applicant: **SANDOZ LTD.**
**Lichtstrasse 35**
**CH-4002 Basel(CH)**
(84) **BE CH DK ES FR GB GR IT LI LU NL SE**

Applicant: **SANDOZ-PATENT-GMBH**
**Humboldtstrasse 3**
**W-7850 Lörrach(DE)**
(84) **DE**

Applicant: **SANDOZ-ERFINDUNGEN Verwaltungsgesellschaft m.b.H.**
**Brunner Strasse 59**
**A-1235 Wien(AT)**
(84) **AT**

(72) Inventor: **Berner, Heinz**
**Geyergasse 2A**
**A-1180 Vienna(AT)**

(54) **[N-(gamma-glutamyl)glycyl] alanine derivatives.**

(57) The compounds of formula I

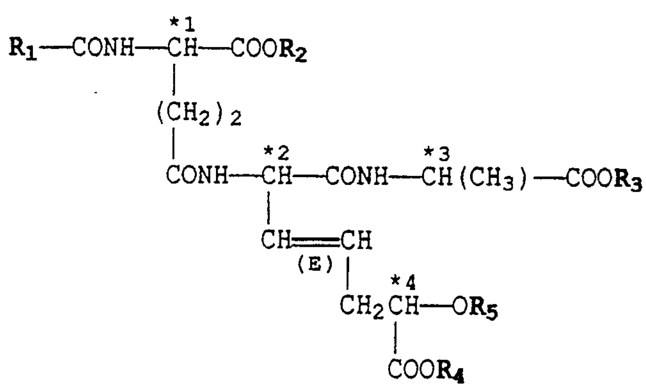

I

wherein
$R_1$ is alkyl or trifluoromethyl,
$R_2$, $R_3$ and $R_4$ independently are hydrogen or a carboxy-protecting group and
$R_5$ is hydrogen or alanyl optionally protected at the amino moiety thereof, with the proviso that when $R_2$, $R_3$ and $R_4$ simultaneously are hydrogen, then $R_5$ is hydrogen,
in free form or as appropriate in salt form,
possess pharmacological activity. In particular they modulate unspecific antimicrobial resistance and possess particularly low pyrogenicity.

They are indicated for use as immunomodulators and antiviral and anti-tumor agents.

They can be prepared by reaction with a glyoxylic acid derivative or reaction with an alanyl derivative, where

EP 0 417 803 A1

appropriate followed by deprotection.

## [N-(γ-GLUTAMYL)GLYCYL]ALANINE DERIVATIVES

The invention concerns a compound of formula I

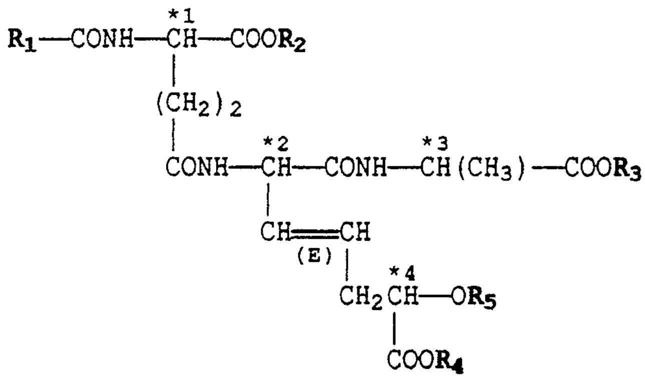

I

wherein
$R_1$ is alkyl or trifluoromethyl,
$R_2$, $R_3$ and $R_4$ independently are hydrogen or a carboxy-protecting group and
$R_5$ is hydrogen or alanyl optionally protected at the amino moiety thereof, with the proviso that when $R_2$, $R_3$ and $R_4$ simultaneously are hydrogen, then $R_5$ is hydrogen,
in free form or as appropriate in salt form.

The carbon atoms marked with an asterisk * in formula I and the α carbon atom in alanyl as substituent $R_5$ are asymmetrically substituted. They can thus have the R or the S configuration.

For convenience these centers of asymmetry are numbered 1 to 4 in formula I after each asterisk. The α carbon atom in alanyl as a substituent $R_5$ is hereafter designated as *5.

The invention comprises a compound of formula I in both racemic and optically pure form. Preferred are the compounds in optically pure form. *1 preferably is in the R configuration. *2 preferably is in the S configuration. *3 preferably is in the R configuration. *5 preferably is in the S configuration.

As appears from formula I the double bond therein has the trans (E) configuration.

$R_1$ preferably is alkyl.

$R_2$, $R_3$ and $R_4$ preferably are a carboxy-protecting group.

$R_5$ preferably is alanyl optionally protected as defined above, preferably unprotected alanyl.

Alkyl preferably is of 1 to 15 carbon atoms, more preferably of 4 to 12, especially of 5 to 11, particularly of 6 to 11 carbon atoms. It preferably is straight-chained.

A carboxy-protecting group preferably is benzyl or alkyl of 1 to 5, preferably of 1 to 4 carbon atoms, especially benzyl.

An amino-protecting group preferably is a hydrolytically splittable group such as formyl or, especially, tert-butyloxycarbonyl.

A subgroup of compounds of formula I is the compounds of formula Ia as defined below, i.e. the compounds of formula I wherein $R_5$ is hydrogen, especially those wherein $R_2$, $R_3$ and $R_4$ are hydrogen. The subgroup of compounds of formula I wherein $R_1$ is as defined above and $R_2$ to $R_5$ are hydrogen is herewith defined as the **compounds Ip.**

A preferred subgroup of compounds of formula I is the compounds of formula I wherein
$R_1$ is alkyl of 5 to 11 carbon atoms,
$R_2$ , $R_3$ and $R_4$ independently are a carboxy-protecting group and
$R_5$ is as defined above under formula I.

A further subgroup of compounds of formula I is the compounds of formula Is

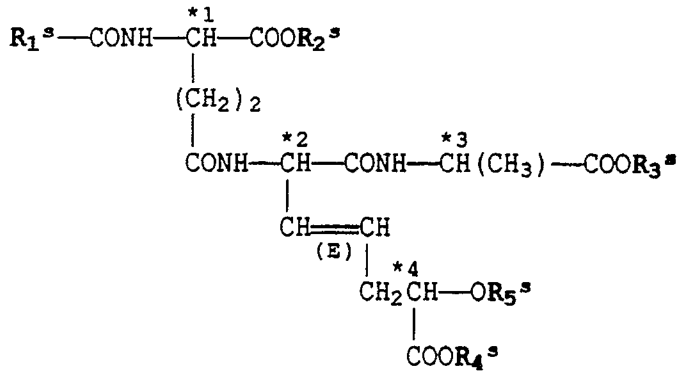

Is

wherein

$R_1^s$ is alkyl of 4 to 12 carbon atoms,

$R_2^s$ is hydrogen, alkyl of 1 to 4 carbon atoms or benzyl,

$R_3^s$ and $R_4^s$ are hydrogen or alkyl of 1 to 4 carbon atoms, and

$R_5^s$ is hydrogen or alanyl optionally protected at the amino moiety thereof by tert-butyloxycarbonyl.

In a subgroup $R_2^s$ is hydrogen, methyl or benzyl.

A compound of formula I can be in free form or appropriately in salt form. For example, when at least one of $R_2$ to $R_4$ is hydrogen it can be in free carboxylic acid or in anionic, e.g. sodium salt form and when $R_5$ is unprotected alanyl, it can be in free base or in acid addition, e.g. hydrochloride salt form. A compound of formula I in free acid or base form can be converted as appropriate into a corresponding compound in anionic or acid addition salt form in conventional manner and vice-versa.

A compound of formula I can be obtained by a process comprising

a) for the preparation of a compound of formula Ia

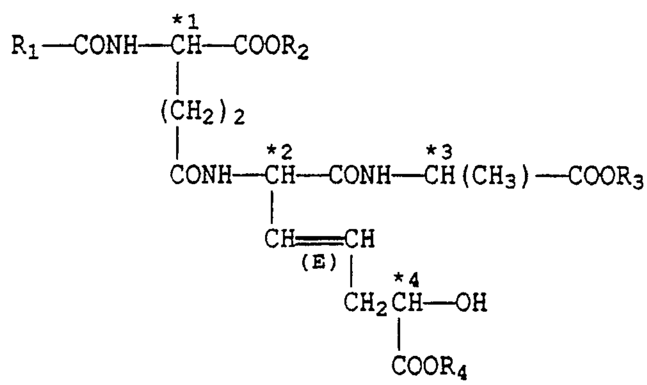

Ia

wherein $R_1$ to $R_4$ are as defined above,

reacting a compound of formula II

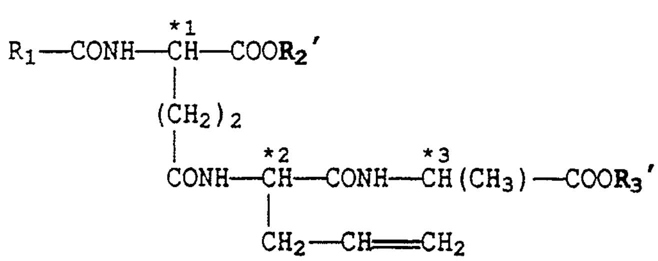

II

wherein

$R_1$ is as defined above and

$R_2'$ and $R_3'$ independently are a carboxy-protecting group,

with a compound of formula III

$OHC-COOR_4'$ III

wherein $R_4'$ is a carboxy-protecting group,

and where appropriate splitting off in one or more steps the carboxy-protecting groups from the resultant compound of formula Ia′

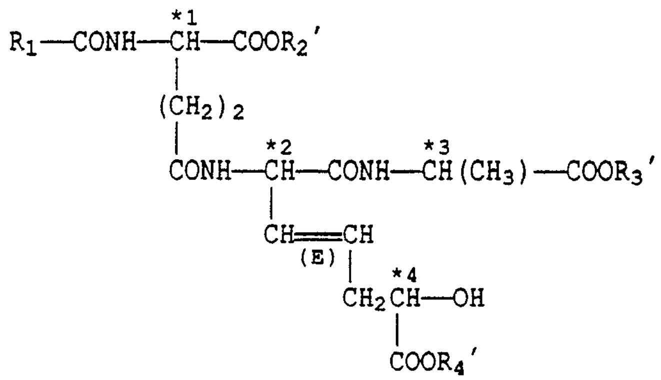

Ia′

wherein $R_1$, $R_2'$, $R_3'$ and $R_4'$ are as defined above; or

b) for the preparation of a compound of formula Ib

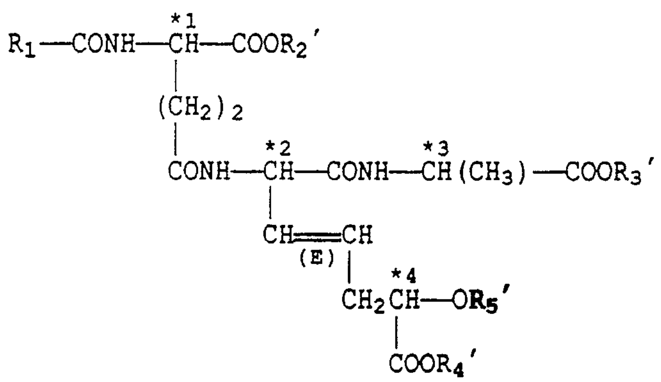

Ib

wherein

$R_1$, $R_2'$, $R_3'$ and $R_4'$ are as defined above and

$R_5'$ is alanyl optionally protected at the amino moiety thereof,

reacting a compound of formula Ia′ as defined above with a compound of formula IV

$$HOOC - \overset{*5}{C}H(CH_3) - NHR_6 \qquad IV$$

wherein $R_6$ is an amino-protecting group,

and where appropriate splitting off the amino-protecting group from the resultant compound of formula Ib′

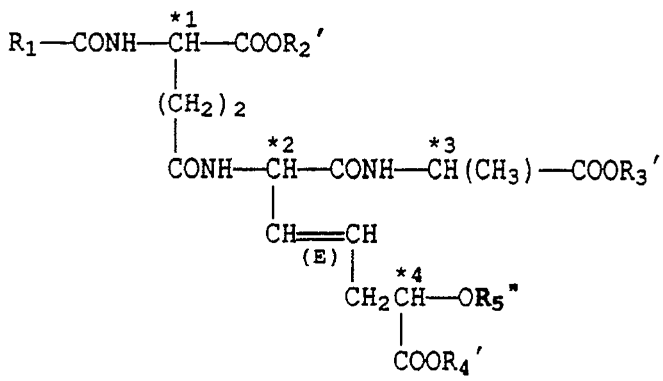

Ib′

wherein

$R_1$, $R_2'$, $R_3'$ and $R_4'$ are as defined above and

$R_5''$ is alanyl protected at the amino moiety thereof, and recovering the resultant compound of formula I in free form or as appropriate in salt form.

The process of the invention can be effected in accordance with known procedures.

Process variant a) is a pericyclic reaction (En reaction). It is e.g. effected by reacting a compound of formula II in a solvent inert under the reaction conditions, e.g. a chlorinated hydrocarbon such as dichloromethane, with a glyoxylic acid ester of formula III. The temperature may vary from about 10° C to about 25° C, it preferably is about room temperature. Conventional carboxy-protecting groups may be used, e.g. benzyl, methyl, ethyl or butyl. Compounds having the double bond in the trans (E) configuration are obtained.

The subsequent optional deprotection step is also effected in conventional manner, e.g. by hydrolysis with a base such as sodium hydroxide. It is effected in one or more, preferably successive steps.

Process variant b) is an esterification. It is e.g. effected by reacting a compound of formula Ia′ in a solvent inert under the reaction conditions, such as a chlorinated hydrocarbon, e.g. dichloromethane, with a protected alanine. Preferably the reaction is effected in the presence of a diimide such as dicyclohexylcarbodiimide. Conventional amino-protecting groups may be used, such as tert-butyloxycarbonyl.

The subsequent optional deprotection step is also effected in conventional manner, e.g. by hydrolysis with an acid such as trifluoroacetic acid.

As indicated above, when $R_5$ is hydrogen the compounds of formula I have at least four centers of asymmetry and when $R_5$ is optionally protected alanyl they have at least five such centers. When the process steps of the invention are effected the configuration remains unchanged, i.e. starting from a compound with the R or, respectively, the S configuration at a particular center of asymmetry, a product with the same configuration at that center is obtained. Compounds produced in a reaction step resulting in the formation of a new center of asymmetry are normally obtained as a mixture of compounds having the R and S configuration at that center, i.e. as a racemate. Such mixtures can be fractionated in conventional manner into the individual diastereoisomers, e.g. chromatographically.

The starting materials can be obtained in conventional manner, e.g., for the compounds of formula II, according to the following reaction scheme:

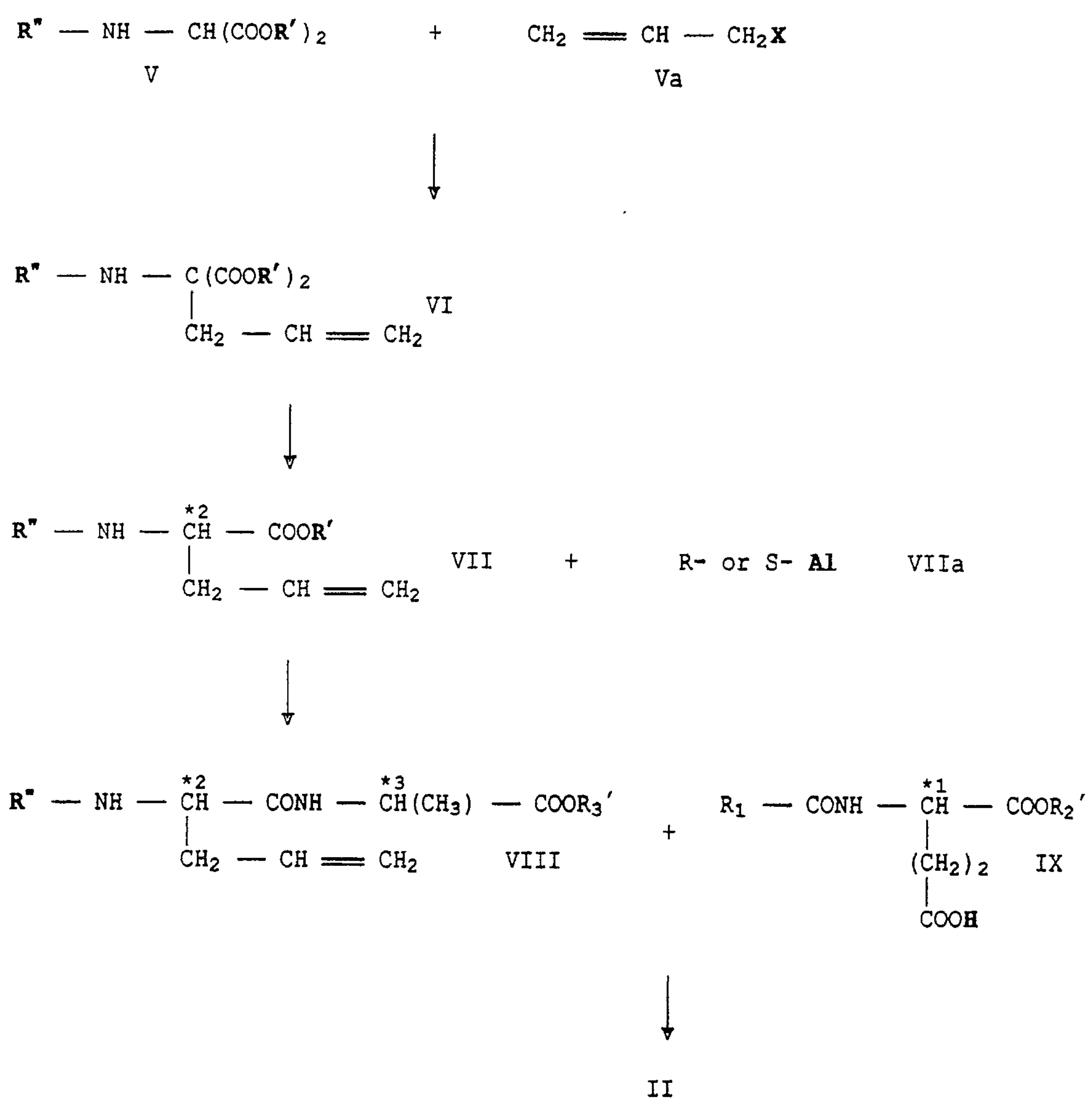


wherein $R_1$, $R_2'$ and $R_3'$ are as defined above; R′ and R″ each are a protecting group; X is a leaving group such as halogen; and Al is alanine or protected alanine.

Thus starting from protected α-aminomalonic acid of formula V, α-alkylation and partial saponification and decarboxylation lead to the racemate of the α-glycine ester of formula VII. This compound is saponified, e.g. at 0 °C with 1N sodium hydroxide, and coupled with an appropriate (R)- or (S)-alanine or -alanine derivative. The resultant diastereoisomeric mixtures are chromatographically fractionated, whereby the configuration at the glycine moiety is determined e.g. by enzymatic hydrolysis. The resultant, sterically homogeneous products of formula VIII (RR, SR, RS and SS) are reacted with the mixed anhydride of formula IX, e.g. the mixed anhydride from N-($R_1$CO)-(R)-glutamic acid benzyl ester and chloroformic acid isobutyl ester, to obtain the diastereoisomers of formula II.

Insofar as its preparation is not specifically described herein, e.g. in the Examples, a compcund used as a starting material is known or can be prepared in conventional manner from known compounds.

The following Examples illustrate the invention but are not limitative. All temperatures are in degrees Centigrade. The abbreviations have the following meaning:
Ala alanyl;
BOC tert-butyloxycarbonyl;
Bu or butyl n-butyl;
Bz benzyl;
DMSO dimethylsulfoxide;
Et ethyl;
Hex n-hexyl;

Me methyl;
na not applicable;
Phe phenyl;
Und n-undecyl.

Where a compound is in optically pure form at a particular center of asymmetry, i.e. when it has either the R or the S configuration at that center but the absolute configuration is not known, this is indicated by the symbol "X" or "Y". Where the configuration at a particular center of asymmetry is racemic this is indicated by the symbol "RS".

**Example 1 : {N-[N′-Heptanoyl-(R)-γ-glutamyl-(α-benzylester)]-(R)-α-[2-(RS)-hydroxypent-4-enoic acid butyl ester-5-yl]glycyl]-(S)-alanine ethyl ester**

[Formula I: $R_1$ = Hex; $R_2$ = Bz; $R_3$ = Et; $R_4$ = Bu; $R_5$ = H; configuration: *1: R; *2: R; *3: S; *4: RS]

[Process variant a)]

To a solution of 1.17 g freshly distilled **glyoxylic acid n-butyl ester** (compound of formula III wherein $R_4'$ is Bu) and 3.12 g tin tetrachloride in 20 ml of absolute methylene chloride is slowly added dropwise at 20° a solution of 1.06 g **{N-[N′-heptanoyl-(R)-γ-glutamyl-(α-benzylester)]-(R)-α-allylglycyl}-(S)-alanine ethyl ester** (compound of formula II wherein $R_1$ = Hex; $R_2'$ = Bz; $R_3'$ = Et; configuration: *1: R; *2: R; *3: S) in 10 ml of absolute methylene chloride. After 3 hours and conventional working-up the mixture is chromatographed over silicagel (eluant: dichloromethane/isopropanol/cyclohexane 3:2:7). The **title compound** is obtained (colourless, amorphous):
$^1$H-NMR (DMSO): 8.4, 8.38 (2d, 1H, NH); 8.22 (d, 1H, NH, 6 Hz); 8.12 (d, 1H, NH, 7 Hz); 7.38 (s, 5H, $C_6H_5$); 5.68 (m, 1H, olefine); 5.5 (m, 1H, olefine); 5.13 (s, 2H, OBZ); 4.92 (bt, 1H, α-H-Gly); 4.21 (quin, 1H, α-H-Ala, 6 Hz); 4.24 (m, 1H, α-H-Gly); 4.05 (m, 4H, OEt and OBu); 1.27 (d, 3H, Et, 6 Hz); 1.18 (t, 3H, OEt); 0.88 (t, 3H, OBu); 0.85 (t, 3H, Hex).

The compound of formula II used as a starting material is obtained in a manner analogous to the compound of formula II for Example 3 (see footnote [2]) in the Table hereunder):
$^1$H-NMR (DMSO): 8.39 (d, 1H, NH, 6 Hz); 8.23 (d, 1H, NH, 6 Hz); 7.98 (d, 1H, NH, 7 Hz); 7.37 (s, 5H, Phe); 5.75 (m, 1H, olefine); 5.12 (s, 2H, OBz); 5.08 (m, 2H, olefine); 4.23 (quin, 1H, α-H-Ala, 6 Hz); 4.25 (m, 1H, α-H-Glu); 4.36 (m, 1H, α-H-Gly); 4.06 (g, 2H, OEt, 6 Hz); 1.28 (d, 3H, $CHCH_3$); 1.17 (t, 3H, OEt); 0.84 (t, 3H, Hex).

The following compounds of formula I are obtained according to process variant a) in a manner analogous to Example 1:

| Example No. | $R_1$ | $R_2$ | $R_3$ | $R_4$ | $R_5$ | Configuration at *1 | *2 | *3 | *4 | *5 | Physicochemical characterization data |
|---|---|---|---|---|---|---|---|---|---|---|---|
| 2[3)] | Hex | Bz | Et | Bu | H | R | S | S | RS | na | M.P. 130-133°; NMR[1)] |
| 3[2)] | Hex | Bz | Me | Bu | H | R | R | R | RS | na | NMR[1)] |
| 4[3)] | Hex | Bz | Me | Bu | H | R | S | R | RS | na | NMR[1)] |
| 5[3)] | Und | Bz | Me | Bu | H | R | S | R | RS | na | NMR[1)] |
| 6[3)] | Und | Bz | Et | Bu | H | R | R | S | RS | na | NMR[1)] |
| 6a | Hex | Me | Me | Me | H | R | S | R | RS | na | NMR[1)] |

1) $^1$H-NMR: Example 2 ($CDCl_3$): 6.95 (m, 2H, NH); 6.64 (d, 1H, NH, 6 Hz); 7.35 (s, 5H, Phe); 5.5, 5.87 (td, 1H, olefine, 12H, 6 Hz); 5.62 (dd, 1H, olefine, 12 Hz, 6 Hz); 5.18 (s, 2H, OBz); 4.95 (m, 1H, α-H-Gly); 4.51, 4.56 (quin, 1H, α-H-Ala); 4.2 (m, 5H, OEt, $OCH_2C_3H_7$, α-H-Glu); 1.41 (t, 3H, OEt); 0.94, 0.95 (t, 3H, OBu); 0.87 (t, 3H, Hex).

Example 3 (DMSO): 5.74 (td, 1H, olefine, 12 Hz, 7 Hz); 5.57 (dd, 1H, olefine, 12 Hz, 7 Hz); 4.86 (m, 1H, α-H-Gly); 4.33 (quin, 1H, α-H-Ala, 6 Hz); 4.34 (m, 1H, α-H-Glu); 4.08 (m, 3H, CHOH, $OCH_2C_3H_7$); 3.64 (s, 3H, OMe); 1.3 (d, 3H, $CHCH_3$, 6 Hz); 0.9 (t, 3H, OBu); 0.84 (t, 3H, Hex).

Example 4 (DMSO): 5.5 (dd, 1H, olefine, 12 Hz, 6 Hz); 5.44 (d, 1H, OH, 5 Hz); 5.12 (s, 2H, OBz); 4.89 (t, 1H, α-H-Gly); 4.72, 4.68 (td, 1H, olefine, 12 Hz, 6 Hz); 4.26 (m, 1H, α-H-Glu); 4.25 (quin, 1H, α-H-Ala, 6 Hz); 4.03 (m, 3H, $OCH_2C_3H_7$, CHOH); 3.62 (s, 3H, OMe); 1.25 (d, 3H, Et, 6 Hz); 1.38 (t, 3H, OBu,); 1.34 (t, 3H, Hex).

Example 5 ($CD_3OD/C_6D_6$ 3:1): 5.9 (m, 1H, olefine); 5.7 (m, 1H, olefine); 5.0 (dd, 1H, α-H-Gly, 7.5 Hz, 4 Hz); 4.48 (m, 1H, α-H-Glu); 4.42 (q, 1H, α-H-Ala, 6.25 Hz); 4.22 (m, 1H, α-H-OH); 4.1 (m, 4H, O-$CH_2$); 5.1 (s, 2H, Bz); 0.90, 0.92 (t, 3H, Und); 1.17, 1.19 (t, 3H, OEt); 1.38, 1.39 (d, 3H, $CH_3$-Ala).

Example 6 ($CD_3OD$): 5.9 (m, 1H, olefine); 5.7 (m, 1H, olefine); 4.99 (dd, 1H, α-H-Gly, 7.5 Hz, 4 Hz); 5.12 (s, 2H, Bz); 4.53 (dd, 1H, α-H-Glu, 10 Hz, 5 Hz); 4.45 (q, 1H, α-H-Ala, 6.25 Hz); 4.22 (m, 1H, α-H-OH); 4.1 (m, 4H, -$OCH_2$-); 3.61, 3.62 (s, 3H, COOMe); 0.90, 0.92 (t, 3H, Und); 1.37, 1.38 (d, 3H, Me, Me-Ala).

Example 6a ($CD_3OD/CD_6D_6$ 3:1): 5.9 (m, 1H, olefine); 5.7 (m, 1H, olefine); 5.02 (dd, 1H, α-H-Gly, 1.5 Hz, 7.5 Hz); 4.46 (q, 1H, α-H-Ala, 7.5 Hz); 4.5 (m, 1H, α-H-Glu); 4.24 (dd, 1H, $CH_2CHOH$, 5 Hz, 7.5 Hz); 3.65 (broad, 9H, $COOCH_3$); 1.38 (d, 3H, $CH_3$-Ala, 7.5 Hz); 1.39 (d, 3H, $CH_3$-Ala, 7.5 Hz); 0.9 (t, 3H, Hex).

2) The starting material **{N-[N'-heptanoyl-(R)-γ-glutamyl-(α-benzylester)]-(R)-α-allylglycyl}-(R)-alanine methyl ester** (compound of formula II wherein $R_1$ = Hex; $R_2'$ = Bz; $R_3'$ = Me; configuration at *1, *2 and *3: R) is obtained as follows: a solution of 1.5 g **(tert-butyloxycarbonyl-(R)-α-allylglycyl)-(R)-alanine methyl ester** (compound of formula VIII wherein R" = BOC; $R_3'$ = Me; configuration at *2 and *3: R) [see C) below] and 950 ml of p-toluenesulfonic acid in 20 ml of 80 % trifluoroacetic acid is kept for 20 to 30 minutes at 20° and thereafter concentrated to dryness under reduced pressure at 50°. After addition of 20 ml of dichloromethane the sulfonate is converted to the base with 1.5 g N-methylmorpholine and the reaction mixture is slowly added dropwise to a mixture of 1.75 g **N-heptanoyl-(R)-glutamic acid-α-benzyl ester**, 0.5 g

N-methylmorpholine and 0.6 g **chloroformic acid isobutyl ester** (mixed anhydride of formula IX) in 20 ml of dichloromethane. After 2 hours working-up is effected in conventional manner and the **product** is chromatographed over silicagel (eluant: chloroform/methanol 100:7):

$^1$H-NMR (DMSO): 8.81 (d, 1H, NH, 6 Hz); 8.21 (d, 1H, NH, 6 Hz); 7.95 (d, 1H, NH, 7 Hz); 7.37 (s, 5H, Phe); 5.13 (s, 2H, OBz); 5.72 (m, 1H, olefine); 5.05 (m, 2H, olefine); 4.26 (quin, 1H, α-H-Ala, 6 Hz); 4.25 (m, 1H, α-H-Glu); 4.36 (m, 1H, α-H-Gly); 3.63 (s, 3H, OMe); 1.29 (d, 3H, $CHCH_3$); 0.86 (t, 3H, Hex).

3) The starting material of formula II is obtained in a manner analogous to 2) above, whereby the resultant compound of formula II has the following $^1$H-NMR:

for Example 2 (DMSO): 8.39 (d, 1H, NH, 6 Hz); 8.23 (d, 1H, NH, 6 Hz); 7.98 (d, 1H, NH, 7 Hz); 7.37 (s, 5H, $C_6H_5$); 5.12 (s, 2H, OBz); 5.75 (m, 1H, olefine); 5.08 (m, 2H, olefine); 4.23 (quin, 1H, α-H-Ala); 4.25 (m, 1H, α-H-Glu); 4.36 (m, 1H, α-H-Gly); 4.06 (q, 2H, OEt, 6 Hz); 1.28 (d, 3H, $CHCH_3$); 1.17 (t, 3H, OEt); 0.84 (t, 3H, Hex);

for Example 4 (DMSO): 8.37 (d, 1H, NH, 6 Hz); 8.21 (d, 1H, NH, 6 Hz); 7.95 (d, 1H, NH, 7 Hz); 7.37 (s, 5H, Phe); 5.12 (s, 2H, OBz); 5.7 (m, 1H, olefine); 5.05 (m, 2H, olefine); 4.27 (quin, 1H, α-H-Ala, 6 Hz); 4.26 (m, 1H, α-H-Glu); 4.38 (m, 1H, α-H-Gly); 3.61 (s, 3H, OMe); 1.25 (d, 3H, $CHCH_3$); 0.86 (t, 3H, Hex);

for Example 5 ($CD_3OD/C_6D_6$ 3:1): 5.08 (m, 1H, olefine); 5.1 (m, 2H, olefine); 5.12 (s, 2H, Bz); 4.5 (m, 2H, α-H-Glu, α-H-Gly); 4.43 (q, 1H, α-H-Ala, 6.25 Hz); 3.62 (s, 3H, $OCH_3$); 1.36 (d, 3H, $CH_3$-Ala, 6.25 Hz); 0.95 (t, 3H, Und-CO);

for Example 6 ($CD_3OD/C_6D_6$ 3:1): 5.8 (m, 1H, olefine); 5.1 (m, 2H, olefine); 5.11 (s, 2H, Bz); 4.5 (m, 2H, α-H-Glu, α-H-Gly); 4.41 (q, 1H, α-H-Ala, 6.25 Hz); 4.07 (q, 2H, OEt); 1.35 (d, 3H, $CH_3$-Ala, 6.25 Hz); 1.16 (t, 3H, OEt); 0.89 (t, 3H, Und-CO).

**Example 7 : {N-[N′-Heptanoyl-(R)-γ-glutamyl]-(R)-α-[2-(RS)-hydroxypent-4-enoic acid-5-yl]glycyl}--(R)-alanine**

[Formula I: $R_1$ = Hex; $R_2$, $R_3$, $R_4$, $R_5$ = H; configuration: *1: R; *2: R; *3: R; *4: RS]

[Process variant a), deprotection step]

270 mg **{N-[N′-Heptanoyl-(R)-γ-glutamyl-(α-benzylester)]-(R)-α-[2-(RS)-hydroxypent-4-enoic acid butyl ester-5-yl]-glycyl}-(R)-alanine methyl ester** (compound of Example 3) is dissolved in a mixture of 10 ml of tetrahydrofuran and 1.2 ml of 1N NaOH and saponified at 0° for 3 hours. The reaction mixture is brought to pH 4.5 with 1N HCl and lyophilised. The residue is chromatographed over HP20 (DAICEL) using a water/acetone gradient (up to 15 % acetone). After renewed lyophilisation the **title compound** is obtained:
$^1$H-NMR ($D_2O$): 5.95 (m, 1H, olefine); 5.66 (dd, 1H, olefine, 12 Hz, 6 Hz); 4.18 (m, 3H, α-H-Ala, α-H-Glu); 1.36, 1.38 (d, 3H, $CHCH_3$, 6 Hz); 0.86 (t, 3H, Hex).

The following compounds of formula I are obtained according to process variant a) (deprotection step) in a manner analogous to Example 7:

| Example No. | $R_1$ | $R_2$ | $R_3$ | $R_4$ | $R_5$ | Configuration at *1 | *2 | *3 | *4 | *5 | Physicochemical characterization data |
|---|---|---|---|---|---|---|---|---|---|---|---|
| 8[2)] | Hex | H | H | H | H | R | S | S | RS | na | NMR[1)] |
| 9[3)] | Hex | H | H | H | H | R | S | R | RS | na | M.P. 213-217°; NMR[1)] |
| 10[4)] | Hex | H | H | H | H | R | R | S | RS | na | - |
| 11[5)] | Und | H | H | H | H | R | S | R | RS | na | - |
| 12[6)] | Und | H | H | H | H | R | R | S | RS | na | - |

1) $^1$H-NMR: Example 8 ($D_2O$): 5.95 (m, 1H, olefine); 5.68 (dd, 1H, olefine, 12 Hz, 6 Hz); 4.19 (m, 2H, α-H-Ala, α-H-Glu); 4.1 (m, 1H, CHOH); 1.36, 1.34 (d, 1H, $CHCH_3$, 6 Hz); 0.88 (t, 3H, Hex).

Example 9 ($D_2O$): 5.89 (td, 1H, olefine, 10 Hz, 6 Hz); 5.67 (dd, olefine, 12 Hz, 6 Hz); 4.18 (m, 2H, α-H-Ala, α-H-Glu); 4.07 (m, 1H, CHOH); 1.35, 1.33 (d, 1H, $CHCH_3$, 6 Hz); 0.88 (s, 3H, Hex).

2) Starting from the compound of Example 2
3) Starting from the compound of Example 4
4) Starting from the compound of Example 1
5) Starting from the compound of Example 5
6) Starting from the compound of Example 6

**Example 13: {N-[N'-Heptanoyl-(R)-γ-glutamyl-(α-benzylester)]-(S)-α-[2-(X)-(tert-butyloxycarbonyl-(S)-alanyloxy)pent-4-enoic acid butyl ester-5-yl]glycyl}-(R)-alanine methyl ester**

and

**{N-[N'-Heptanoyl-(R)-γ-glutamyl-(α-benzylester)]-(S)-α-[2-(Y)-(tert-butyloxycarbonyl-(S)-alanyloxy)pent-4-enoic acid butyl ester-5-yl]glycyl}-(R)-alanine methyl ester**

[Formula I: $R_1$ = Hex; $R_2$ = Bz; $R_3$ = Me; $R_4$ = Bu; $R_5$ = BOC-(S)-alanyl;
configuration: *1: R; *2: S; *3: R; *4: X and, respectively, Y; *5: S]

[Process variant b)]

To a solution of 0.99 g **{N-[N'-heptanoyl-(R)-γ-glutamyl-(α-benzylester)]-(S)-α-[2-(RS)-hydroxypent-4-enoic acid butyl ester-5-yl]-glycyl}-(R)-alanine methyl ester** (compound of Example 4), 0.28 g **BOC-(S)-alanine** and 15 mg 4-dimethylaminopyridine (Steglich base) in 15 ml of absolute dichloromethane is added at 0° in portions 0.31 g dicyclohexylcarbodiimide. After 15 hours at 15° the precipitated urea is filtered off, the solution is concentrated and the diastereoisomers are chromatographically fractionated over silicagel (eluant: cyclohexane/isopropanol/dichloromethane 10:1:3). The **title compounds** are obtained:

(X)-diastereoisomer: $[\alpha]_D^{20}$ = +28.5° (c = 1 in methanol);
M.P. 121-122°;
$^1$H-NMR ($CD_3OD/C_6D_6$ 3:1): 5.9 (m, 1H, olefine); 5.7 (m, 1H, olefine); 5.1 (s, 2H, Bz); 5.02 (d, 1H, α-H-Gly, 6.25 Hz); 5.07 (t, 1H, $CH_2CHOCO$, 5 Hz); 4.52 (dd, 1H, α-H-Glu, 8.75 Hz, 5 Hz); 4.45 (q, 1H, α-H-Ala, 6.25 Hz); 4.32 (m, 1H, α-H-Ala); 4.08 (t, 2H, COOBu); 3.60 (s, 3H, OMe); 1.37 (d, 6H, 2 x Me-Ala, 6.25 Hz); 0.87 (t, 3H, Hex);

(Y)-diastereoisomer: $[\alpha]_D^{20}$ = +16.5° (c = 1 in methanol);
$^1$H-NMR ($CD_3OD/C_6D_6$ 3:1): 5.9 (m, 1H, olefine); 5.7 (m, 1H, olefine); 5.16 (s, 2H, Bz); 4.94 (d, 1H, α-H-Gly, 6.25 Hz); 5.08 (t, 1H, $-CH_2CHOCO$, 5 Hz); 4.42 (m, 2H, α-H-Ala, α-H-Glu); 4.2 (q, 1H, α-H-Ala, 6.25 Hz); 4.12 (t, 2H, COOBu); 3.69 (s, 3H, OMe); 1.4 (d, 3H, Me-Ala, 6.25 Hz); 1.38 (d, 3H, Me-Ala, 6.25 Hz); 0.95 (t, 3H, OBu); 0.90 (t, 3H, Hex).

The following compounds of formula I are obtained according to process variant b) in a manner analogous to Example 13:

| Example No. | $R_1$ | $R_2$ | $R_3$ | $R_4$ | $R_5$ | Configuration at *1 | *2 | *3 | *4 | *5 | Physicochemical characterization data |
|---|---|---|---|---|---|---|---|---|---|---|---|
| 14a[2] | Hex | Bz | Et | Bu | BOC-(S)-Ala | R | S | S | X | S | NMR[1] |
| 14b[2] | Hex | Bz | Et | Bu | BOC-(S)-Ala | R | S | S | Y | S | NMR[1] |
| 14c[3] | Hex | Me | Me | Me | BOC-(R)-Ala | R | S | R | RS | S | NMR[1] |

[1] $^1$H-NMR: Example 14a ($CD_3OD/C_6D_6$ 3:1): 5.9 (m, 1H, olefine); 5.7 (m, 1H, olefine); 5.07 (m, 1H, $CH_2CHOCO$); 5.0 (d, 1H, α-H-Gly, 7.5 Hz); 5.12 (s, 2H, Bz); 4.53 (dd, 1H, α-H-Glu, 5 Hz, 10 Hz); 4.4 (q, 1H, α-H-Ala, 7.5 Hz); 4.3 (m, 1H, α-H-Ala-BOC); 4.1 (m, 4H, OBu, OEt); 1.4 (d, 6H, 2 x Me-Ala, 7.5 Hz); 1.19 (t, 3H, OEt); 0.95 (m, 6H, Hex, OBu).

Example 14b ($CD_3OD/C_6D_6$ 3:1): 5.9 (td, 1H, olefine, 7.5 Hz, 15 Hz); 5.69 (dd, 1H, olefine, 5 Hz, 15 Hz); 5.08 (m, 1H, $CH_2CHOCO$); 5.01 (dd, 1H, α-H-Gly, 5 Hz, 1 Hz); 4.55 (dd, 1H, α-H-Glu, 5 Hz, 10 Hz); 4.4 (q, 1H, α-H-Ala, 7.5 Hz); 4.34 (q, 1H, α-H-Ala-BOC); 4.1 (m, 4H, OEt, OBu); 1.38 (d, 6H, 2 x Me-Ala, 7.5 Hz); 1.18 (t, 3H, OEt); 0.95 (m, 6H, Hex, OBu).

Example 14c ($CD_3OD/C_6D_6$ 3:1): 5.9 (m, 2H, olefine); 5.1 (t, 1H, $CH_2CHOCO$, 5 Hz); 5.03 (dd, 1H, α-H-Gly, 1.5 Hz, 7.5 Hz); 4.45 (q, 1H, α-H-Ala, 7.5 Hz); 4.5 (m, 1H, α-H-Glu); 4.3 (m, 1H, α-H-Ala-BOC); 3.63 (broad, 9H, 3 x $COOCH_3$); 1.45 (broad, 9H, t-Bu); 1.38 (d, 6H, 2 x $CH_3$-Ala, 7.5 Hz); 0.9 (t, 3H, Hex).

[2] Starting from the compound of Example 2

[3] Starting from the compound of Example 6a, without fractionation of the diastereoisomers

Example 15 : {N-[N′-Heptanoyl-(R)-γ-glutamyl-(α-benzylester)]-(S)-α-[2-(Y)-((S)-alanyloxy)pent-4-enoic acid butyl ester-5-yl]glycyl}-(R)-alanine methyl ester

[Formula I: $R_1$ = Hex; $R_2$ = Bz; $R_3$ = Me; $R_4$ = Bu; $R_5$ = (S)-Ala; configuration *1: R; *2: S; *3: R; *4: Y; *5: S]

[Process variant b), deprotection step]

200 mg **{N-[N′-heptanoyl-(R)-γ-glutamyl-(α-benzylester)]-(S)-α-[2-(Y)-(tert-butyloxycarbonyl-(S)-alanyloxy)pent-4-enoic acid butyl ester-5-yl]glycyl}-(R)-alanine methyl ester** (compound of Example 13, (Y)-diastereoisomer) is added at 0° in portions to 5 ml of trifluoroacetic acid. After 3 minutes the mixture is evaporated to dryness under reduced pressure, the residue is taken up in dichloromethane and this solution washed with 10 % sodium bicarbonate solution. The organic phase is dried over sodium sulfate and again evaporated to dryness, the residue is taken up in dioxane/5 % HCl and lyophilised. The **title compound** (in hydrochloride acid addition salt form) is obtained:
$[\alpha]_D^{20}$ = +23.6° (c = 1 in methanol);
M.P. 85-87°;
$^1$H-NMR ($CD_3OD/C_6D_6$ 3:1): 5.83 (td, 1H, olefine, 7.5 Hz, 15 Hz); 5.7 (dd, 1H, olefine, 5 Hz, 15 Hz); 5.14 (dd, 1H, $CH_2CHOCO$, 4 Hz, 7.5 Hz); 5.0 (d, 1H, α-H-Gly, 6.25 Hz); 4.54 (dd, 1H, α-H-Glu, 5 Hz, 3.75 Hz); 4.4 (q, 1H, α-H-Ala, 6.25 Hz); 4.07 (t, 2H, OBu); 3.69 (q, 1H, α-H-Ala-$NH_2$); 3.62 (s, 3H, OMe); 1.4, 1.34 (2 x d, Me-Ala, 6.25 Hz); 0.88, 0.90 (2 x t, 2 x 3H, Hex, OBu).

The following compounds of formula I are obtained according to process variant b), deprotection step in a manner analogous to Example 15:

| Example No. | $R_1$ | $R_2$ | $R_3$ | $R_4$ | $R_5$ | Configuration at *1 | *2 | *3 | *4 | *5 | Physicochemical characterization data |
|---|---|---|---|---|---|---|---|---|---|---|---|
| **16**[2)] | **Hex** | **Bz** | **Me** | **Bu** | **(S)-Ala** | R | S | R | X | S | Hydrochloride: $[\alpha]_D^{20}$ = +38.4° (c = 1 in methanol); NMR[1)] |
| **17**[3)] | **Hex** | **Bz** | **Et** | **Bu** | **(S)-Ala** | R | S | S | Y | S | NMR[1)] |

1) $^1$H-NMR: Example 16 ($CD_3OD/C_6D_6$ 3:1): 5.82 (td, 1H, olefine, 7.5 Hz, 15 Hz); 5.72 (dd, 1H, olefine, 5 Hz, 15 Hz); 5.08 (dd, 1H, $CH_2CHOCO$, 4Hz, 5.25 Hz); 5.04 (d, 1H, α-H-Gly, 6.25 Hz); 4.54 (dd, 1H, α-H-Glu, 5 Hz, 3.7 Hz); 4.4 (q, 1H, α-H-Ala, 6.25 Hz); 4.09 (t, 2H, OBu); 3.62 (s, 3H, OMe); 3.57 (q, 1H, α-H-Ala- $NH_2$); 1.3, 1.35 (2 x d, Me, Ala, 6.25 Hz); 0.88, 0.90 (2 x t, 2 x 3H, Hex, OBu);

Example 17 ($CD_3OD/C_6D_6$ 3:1): 5.89 (td, 1H, olefine, 7.5 Hz, 15 Hz); 5.71 (dd, 1H, olefine, 5 Hz, 15 Hz); 5.12 (s, 2H, Bz); 5.05 (m, 2H, $CH_2CHOCO$, α-H-Gly); 4.55 (dd, 1H, α-H-Glu, 5 Hz, 10 Hz); 4.42 (q, 1H, α-H-Ala, 6.25 Hz); 4.1 [m, 4H, $COO(CH_2)_4CH_3$, COOEt]; 3.58 (m, 1H, α-H-Ala-$NH_2$); 1.37, 1.32 (2 x d, 2 x Me-Ala, 6.25 Hz); 1.17 (t, 3H, EtOCO); 0.95 (m, 6H, Hex, BuCO).

2) Starting from the compound of Example 13, (X)-diastereoisomer.

3) Starting from the compound of Example 14b.

**Earlier intermediates** are obtained e.g. as described hereunder for representative compounds:

A) **2-tert-Butyloxycarbonylamino-2-allylmalonic acid diethyl ester**

[Formula VI: R′ = Et; R″ = BOC]

Following alcoholate formation from a solution of 1.8 g sodium in 100 ml of absolute ethanol, a solution of 20.5 g **2-tert-butyloxycarbonylaminomalonic acid diethyl ester** (compound of formula V wherein R′ = Et; R″ = BOC) in 20 ml of ethanol is added dropwise. 9 g **allyl bromide** is then added and the reaction mixture is heated for 4 hours under refluxing. The precipitated sodium bromide is filtered off and the solution concentrated under reduced pressure and worked-up in conventional manner. The **title compound** is obtained as a crude product which can be used without further purification. A sample is chromatographed over silicagel (eluant: hexane/acetic acid ethyl ester 4:1) for characterization:
$^1$H-NMR ($CDCl_3$): 5.9 (m, 1H, NH); 5.65 (m, 1H, olefine); 5.13 (m, 2H, olefine); 4.25 (m, 4H, OEt); 3.04 (d, 2H, $CH_2=CHCH_2$, 7 Hz); 1.44 [s, 9H, $(CH_3)_3C$]; 1.26 (t, 6H, OEt)

B) **tert-Butyloxycarbonyl-(RS)-α-allylglycine ethyl ester**

[Formula VII: R′ = Et; R″ = BOC; configuration at *2: RS]

14.5 g **title compound** from step A) above is taken up in a solution of 30 ml ethanol and 46 ml 1N sodium hydroxide and kept at 20° for 16 hours. The ethanol is then separated fom the reaction mixture under reduced pressure, the mixture is brought to pH 3.5 with 1N hydrochloric acid and extracted with acetic acid ethyl ester. After conventional working-up a product is obtained which is submitted to decarboxylation in xylene (2 hours under refluxing) without further purification. The mixture is concentrated to dryness under reduced pressure and filtered over a short silicagel column (eluent: hexane/acetic acid ethyl ester 6:1). The **title compound** is obtained:
$^1$H-NMR ($CDCl_3$): 5.65 (m, 1H, olefine); 5.12 (m, 3H, NH, olefine); 4.35 (m, 1H, H); 4.2 (q, 2H, OEt); 2.53 (m, 2H, b-H); 1.45 (s, 9H, $(CH_3)_3C$]; 1.28 (t, 3H, OEt).

C) **(tert-Butyloxycarbonyl-(R)-α-allylglycyl)-(R)-alanine methyl ester** and **(tert-butyloxycarbonyl-(S)-α-allylglycyl)-(R)-alanine methyl ester**

[Formula VIII: R″ = BOC; $R_3$′ = Me; configuration: *2: R and, respectively, S; *3: R]

To a solution of 4.3 g **tert-butyloxycarbonyl-(RS)-α-allylglycine** [saponified title compound of step B) above], 2.75 g **(R)-alanine methyl ester** and 2 g N-methylmorpholine in 100 ml of dichloromethane is added in portions at 20° 4.1 g dicyclohexylcarbodiimide. After 24 hours the dicyclohexylurea is filtered off, the solution concentrated to dryness and the residue chromatographed over silicagel (eluant: hexane/acetic acid ethyl ester 5:1). The two diastereoisomeric **title compounds** are obtained:
R-Diastereoisomer: $[\alpha]_D^{20}$ = +17.4° (c = 1 in methanol);
$^1$H-NMR($CDCl_3$) : 6.72 (d, 1H, NH, 7.5 Hz); 5.78 (m, 1H, olefine); 5.15 (m, 2H, olefine); 4.62 (quin, 1H, α-H-Ala, 7.2 Hz); 4.2 (q, 1H, α-H-Gly, 7.2 Hz); 3.8 (s, 3H, OMe); 2.55 (m, 2H, allyl); 1.5 [s, 9H, $(CH_3)_3C$]; 1.4 (d, 3H, $CH_3CH$);
S-Diastereoisomer: $[\alpha]_D^{20}$ = +13.9° (c = 1 in methanol);
1H-NMR ($CDCl_3$) : 6.75 (d, 1H, NH, 7.5 Hz); 5.75 (m, 1H, olefine); 5.18 (m, 2H, olefine); 4.6 (quin, 1H, α-H-Ala, 7.5 Hz); 4.2 (q, 1H, α-H-Gly, 7.2 Hz); 7.76 (s, 3H, OMe); 2.52 (m, 2H, allyl); 1.45 [s, 9H, $(CH_3)_3C$]; 1.4 (d, 3H, $CH_3CH$).

C′) **(tert-Butyloxycarbonyl-(R)-α-allylglycyl)-(S)-alanine ethyl ester and (tert-butyloxycarbonyl-(S)-α-**

allylglycyl)-(S)-alanine ethyl ester

[Formula VIII: $R''$ = BOC; $R_3'$ = Et; configuration: *2: R and, respectively, S; *3: S]

The **title compounds** are obtained in a manner analogous to step C) above, using **(S)-alanine ethyl ester** in place of (R)-alanine methyl ester:
R-Diastereoisomer: $[\alpha]_D^{20}$ = +12.1° (c = 1 in methanol);
$^1$H-NMR ($CDCl_3$): 6.78 (d, 1H, NH, 7.5 Hz); 5.78 (m, 1H, olefine); 5.15 (m, 2H, olefine); 4.57 (quin, 1H, α-H-Ala, 6.25 Hz); 4.2 (q, 2H, OEt, 7.5 Hz); 4.2 (m, 1H, α-H-Gly); 2.52 (m, 2H, allyl); 1.45 [s, 9H, $(CH_3)_3C$]; 1.4 (d, 3H, $CH_3CH$, 6.25 Hz); 1.26 (t, 3H, OEt, 7.5 Hz);
S-Diastereoisomer: $[\alpha]_D^{20}$ = + 24.3° (c = 1 in methanol);
$^1$H-NMR ($CDCl_3$): 6.8 (d, 1H, NH, 7.5 Hz); 5.78 (m, 1H, olefine); 5.15 (m, 2H, olefine); 4.55 (quin, 1H, α-H-Ala, 6.52 Hz); 4.2 (q, 2H, OEt, 7.5 Hz); 4.2 (m, 1H, α-H-Gly); 2.5 (m, 2H, allyl); 1.45 [s, 9H, $(CH_3)_3C$]; 1.4 (d, 3H, $CH_3CH$, 6.25 Hz); 1.26 (t, 3H, OEt, 7.5 Hz).

The compounds of formula I in free form or as appropriate in pharmaceutically acceptable salt form, hereinafter briefly designated "the compounds of the invention", possess pharmacological activity. They are thus indicated for use as pharmaceuticals.

In particular they modulate unspecific antimicrobial resistance and possess immunomodulating, antiviral and anti-tumor activity and further have a particularly reduced degree of pyrogenicity.

The activities may e.g. be determined in the following test methods:

### 1. Pyrogenicity:

Testing is effected in accordance with the US Pharmacopeia 1985 (USP XXI) following intravenous administration of test substance at various concentrations to groups of 3 rabbits. A substance is considered as pyrogenic when the sum of the body temperature increases in 3 animals within 3 hours after injection is clearly above 1.5° C.

No pyrogenicity is found with the compounds of the invention up to dosages of about 500 µg/kg to 1 mg/kg body weight.

### 2. Modulation of unspecific antimicrobial resistance:

Enhancement of unspecific resistance against microbial septicemia in neutropenic mice

The test method is essentially as described in GB 2'179'945 A and DE 37'30'905.

The compounds of the invention exert after repeated parenteral and/or peroral administration (once daily for 3 days) significant (> 80 %) increases in survival rate at a dosage of from about 12.5 mg/kg to about 50 mg/kg.

### 3. Immunomodulating activity:

Induction of colony-stimulating factors (CSFs)

The test method is essentially as described in GB 2'179'945 A and DE 37'30'905.

In this test method the compounds of the invention have high CSF activity-inducing effect upon single or repeated parenteral and/or oral administration in mice in dosages of from about 0.2 mg/kg to about 50 mg/kg.

### 4. Antitumor activity

4.1. Induction of tumor-necrosis factor (TNF)

TNF induction is determined in bone marrow cell cultures of mice. Bone marrow cells from $BDF_1$ mice are cultured for 8-10 days in Teflon$^R$ bags in proliferation medium [DMEM H21 + 10 % fetal calf serum + 5 % horse serum + 15 % L 929 cell supernatant (= CSF) + penicillin 10 U/ml + streptomycin 100 µg/ml]. The cells are then washed by centrifugation and diluted to 1 x $10^6$ cells/ml in the induction medium (RPMI 1640 + 5 % fetal calf serum + penicillin 10 U/ml + streptomycin 100 µg/ml + 1 % glutamine); 1 ml/well aliquots are distributed into 24 well Costar plates and incubation is effected for 24 hours at 37° C with 5 % $CO_2$ with and without recombinant murine ( rm ) γ-interferon (100 U/ml). The cell cultures are then washed twice with induction medium devoid of fetal calf serum and incubated for 4 hours with the test substances diluted in the same medium at a concentration of from 0.1 µg/ml to 100 µg/ml (dilution steps 1:10). The supernatants are collected and either kept at -70° C or directly investigated for TNF activity [T.J. Sayers et al., J. Immunol . 136 (1987) 2935-2940].

4.2. Tumor-necrosis factor (TNF) assay

The test method is essentially as described in DE 37'30'905.

In each assay a TNF standard (recombinant murine TNF from Genzyme) is also used, at an initial concentration of 1000 U/ml. Results expressed in international human TNF units relate to comparisons with human (recombinant DNA) TNF code number 86/659 from WHO International Laboratory for Biological Standards, Herdfordshire, EN 6 3QG.

In this test the compounds of the invention show at concentrations of from about 0.1 µg/ml to about 10 µg/ml no induction of TNF-α release from not preactivated macrophages (interferon-γ), but low amounts (10-30 units/ml) are induced in preactivated macrophages at concentrations of ≧ 10 µg/ml. The compounds of the invention thus have a desirably low endotoxic potential coupled with macrophage activation for immune response.

4.3. Induction of macrophage toxicity against tumor cells (P 815)

In a system where elicited, adherent mouse macrophages are cultured with tumor cells, lipopolysaccharides or immunostimulant substances can activate the macrophages, which then lyse the tumor cells or inhibit their proliferation. This activation can be further enhanced by addition of γ-interferon. The number of surviving cells can be determined by measurement of $^3$H-thymidine incorporation after 24 hours since only these cells are able to incorporate $^3$H-thymidine. A comparison with a negative control and with a lipopolysaccharide-positive control indicates the lytic or cytotoxic activity of a given concentration of test substance in %.

The compounds of the invention possess at a concentration of from about 1 µg/ml to about 10 µg/ml lytic activity of more than 80 % against tumor cells, in combination with 5 or 10 units rm γ-interferon. This in vitro effect is indicative of an interesting anti-tumor activity.

4.4. B16F1 mouse melanoma modell

The test method is essentially as described in GB 2'179'945 A and DE 37'30'905.

The test substances are administered in physiological saline solution parenterally (i.p., i.v., s.c.) or orally. For the assay of prophylactic activity treatment is effected repeatedly on days -6 to -1 prior to tumor inoculation, and for assaying therapeutic activity on days +3 to + 15 at various intervalls after tumor implantation.

The compounds of the invention produce significant reductions in metastase formation in the lungs of test animals after both parenteral and oral administration at a dosage of from about 10 µg/kg to about 50 mg/kg.

**5. Antiviral activity**

Herpes infection in mice :

The test method is essentially as described in GB 2'179'945 A and DE 37'30'905.

The compounds of the invention clearly improve the course of disease, its duration and the rate of survival following experimental HSV-1 infection as compared with untreated controls upon repeated administration between days 0 or -1 and +6.

## 6. Endotoxicity

### 6.1. Procoagulant activity (PCA)

Although no endotoxic activity was detected by the Limulus-amoebocyte lysate test, procoagulant activity ( PCA ) was investigated as a second very sensitive indicator for endotoxicity and for the inhibitory properties of the compounds of the invention.

#### 6.1.1. In vitro

After stimulation with lipopolysaccharide human endothelial cells produce PCA, measured as a decrease in the time required for plasma coagulation.

Macrophages following treatment with lipopolysaccharide and peritoneal leucocytes (obtained from rabbits) following generalized Schwartzman reaction also have this effect on recalcified plasma.

In a test setup a) designed to investigate the effect on lipopolysaccharide-induced PCA, peritoneal macrophages from $B_6D_2F_1$-mice stimulated with thioglycolate are treated overnight with lipopolysaccharide either alone or together with test substance in DMEM medium devoid of fetal calf serum.

In a test setup b), such mouse macrophages are treated for 24 hours with lipopolysaccharide or, respectively, test substance and following a change of medium are stimulated overnight with lipopolysaccharide.

The coagulation time of recalcified human control plasma following addition of repeatedly frozen and ultrasonicated macrophage suspension is determined using the Häckchen method.

The addition of the compounds of the invention reduces the PCA triggered by lipopolysaccharide below the level of the control (increase in duration of coagulation). Pretreatment with a compound of the invention also leads to a reduction in PCA.

#### 6.1.2. In vivo

The effect is determined in mouse peritoneal leucocytes after pretreatment with test substance as follows: $B_6D_2F_1$-mice are treated on days 1, 2 and 3 with lipopolysaccharide or, respectively, test substance, administered intraperitoneally. On day 3 all animals additionally receive 1.5 ml of thioglycolate i.p. On day 6 peritoneal macrophages are recovered, the sample from each animal is brought to $1 \times 10^6$ cells/ml and stimulated for 24 hours with LPS in DMEM medium devoid of fetal calf serum. The PCA of these cells is determined as described above.

Pretreatment with LPS or with the compounds of the invention leads to markedly reduced PCA. An analogous finding is made using rabbits.

### 6.2. Induction of endotoxin shock in the mouse

The test method is essentially as described in GB 2'179'945 A and DE 37'30'905.

For the compound of Example 1 an $LD_{50}$ of 12 mg/kg is found.

The compounds of the invention are therefore indicated for use in the prevention and treatment of conditions requiring immunomodulation, e.g. the modulation of unspecific antimicrobial resistance, and as antiviral and anti-tumor agents.

For these indications the appropriate dosage will, of course, vary depending upon, for example, the host, the mode of administration and the nature and severity of the condition being treated. However, in

general, satisfactory results are indicated to be obtained systemically at daily dosages of from about 1.5 µg/kg to about 100 mg/kg animal body weight. An indicated daily dosage is in the range of from about 0.1 mg to about 7.0 mg, conveniently administered, for example, in divided doses up to four times a day or in retard form.

The compounds of the invention may be administered by any conventional route, in particular enterally, e.g. orally, e.g. in the form of tablets or capsules, or in the form of injectable solutions.

Pharmaceutical compositions comprising a compound of the invention in association with at least one pharmaceutically acceptable carrier or diluent may be manufactured in conventional manner by mixing with a pharmaceutically acceptable carrier or diluent and are part of the present invention. Unit dosage forms contain, for example, from about 0.025 mg to about 35 mg of active substance.

Preferred in the above indications are the compounds of Examples 15 and 16, i.e.

- {N-[N′-heptanoyl-(R)-γ-glutamyl-(α-benzylester)]-(S)-α-[2-(Y)-((S)-alanyloxy)pent-4-enoic acid butyl ester-5-yl]glycyl}-(R)-alanine methyl ester and
- {N-[N′-Heptanoyl-(R)-γ-glutamyl-(α-benzylester)]-(S)-α-[2-(X)-((S)-alanyloxy)pent-4-enoic acid butyl ester-5-yl]glycyl}-(R)-alanine methyl ester,

preferably in hydrochloride salt form.

The invention also comprises the compounds of the invention for use as pharmaceuticals, especially for use in the prevention and treatment of conditions requiring immunomodulation, e.g. the modulation of unspecific antimicrobial resistance, and as antiviral and anti-tumor agents.

The invention further comprises a method for the treatment of conditions requiring immunomodulation, e.g. the modulation of unspecific antimicrobial resistance, and of conditions requiring antiviral and anti-tumor therapy, comprising administering a therapeutically effective amount of a compound of the invention to a patient in need of such treatment.

## Claims

1. A compound of formula I

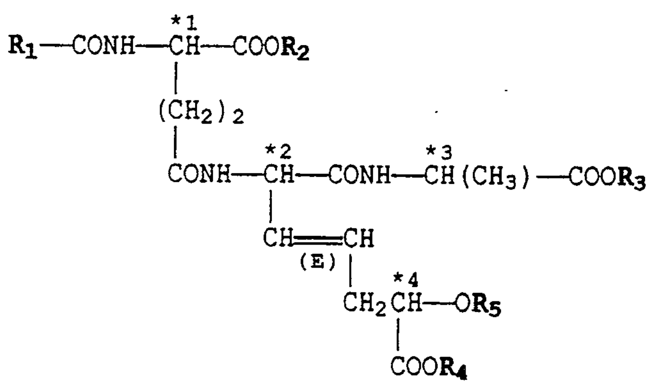

I

wherein

$R_1$ is alkyl or trifluoromethyl,

$R_2$, $R_3$ and $R_4$ independently are hydrogen or a carboxy-protecting group and

$R_5$ is hydrogen or alanyl optionally protected at the amino moiety thereof, with the proviso that when $R_2$, $R_3$ and $R_4$ simultaneously are hydrogen, then $R_5$ is hydrogen,

in free form or as appropriate in salt form.

2. A compound according to claim 1 wherein $R_1$ is as defined in claim 1 and $R_2$ to $R_5$ are hydrogen.

3. A compound according to claim 1 of formula Is

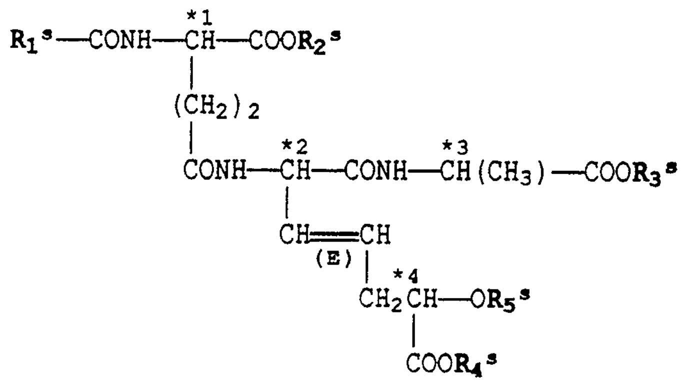

Is

wherein
$R_1{}^s$ is alkyl of 4 to 12 carbon atoms,
$R_2{}^s$ is hydrogen, alkyl of 1 to 4 carbon atoms or benzyl,
$R_3{}^s$ and $R_4{}^s$ are hydrogen or alkyl of 1 to 4 carbon atoms, and
$R_5{}^s$ is hydrogen or alanyl optionally protected at the amino moiety thereof by tert-butyloxycarbonyl,
in free form or as appropriate in salt form.

4. The compound according to claim 1 wherein $R_1$ to $R_5$ respectively are Hex, Bz, Me, Bu and alanyl and the configuration at carbon atoms *1 to *5 respectively is R, S, R, Y and S.

5. The compound according to claim 1 wherein $R_1$ to $R_5$ respectively are Hex, Bz, Me, Bu and alanyl and the configuration at carbon atoms *1 to *5 respectively is R, S, R, X and S.

6. A process for the preparation of a compound according to claim 1 of formula Ia

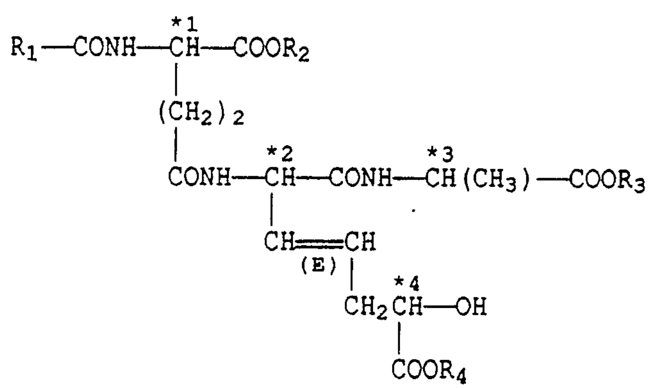

Ia

wherein $R_1$ to $R_4$ are as defined in claim 1,
comprising reacting a compound of formula II

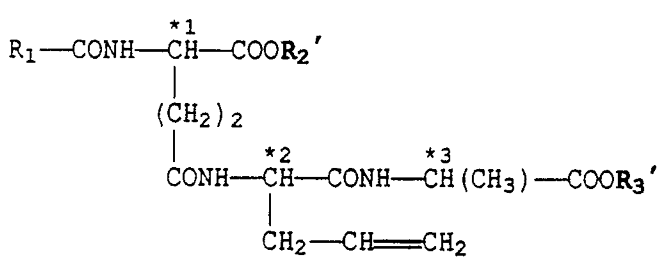

II

wherein
$R_1$ is as defined in claim 1 and
$R_2{}'$ and $R_3{}'$ independently are a carboxy-protecting group,
with a compound of formula III
OHC - COO$R_4{}'$ III
wherein $R_4{}'$ is a carboxy-protecting group,

and where appropriate splitting off in one or more steps the carboxy-protecting groups from the resultant compound of formula Ia′

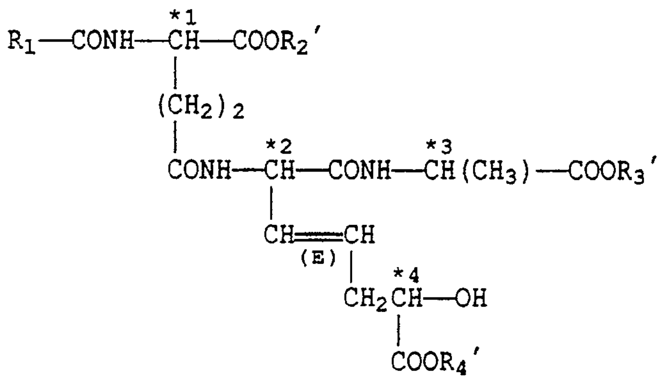

Ia′

wherein

$R_1$ is as defined in claim 1 and

$R_2'$, $R_3'$, and $R_4'$, are as defined in this claim,

and recovering the resultant compound of formula Ia in free form or as appropriate in salt form.

7. A process for the preparation of a compound according to claim 1 of formula Ib

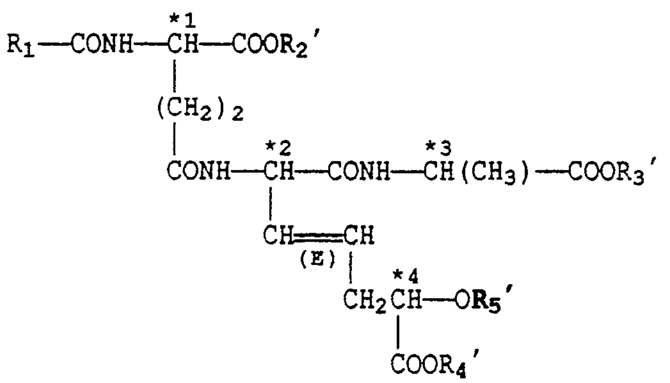

Ib

wherein

$R_1$ is as defined in claim 1,

$R_2'$, $R_3'$, and $R_4'$ are as defined in claim 6 and

$R_5'$ is alanyl optionally protected at the amino moiety thereof,

comprising reacting a compound of formula Ia′ as defined in claim 6 with a compound of formula IV

$$HOOC - \overset{*5}{C}H(CH_3) - NHR_6 \qquad IV$$

wherein $R_6$ is an amino-protecting group,

and where appropriate splitting off the amino-protecting group from the resultant compound of formula Ib′

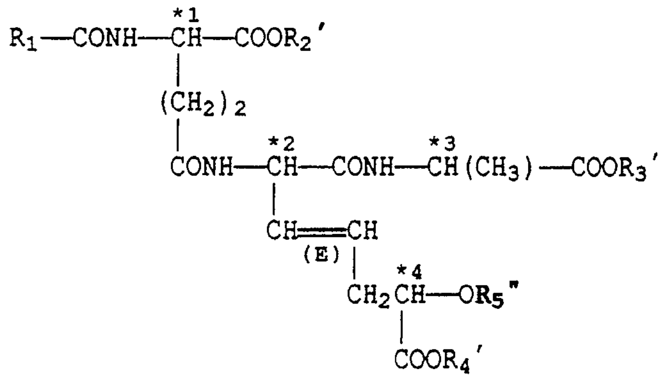

Ib′

wherein
$R_1$ is as defined in claim 1,
$R_2'$, $R_3'$, and $R_4'$ are as defined in claim 6 and
$R5''$ is alanyl protected at the amino moiety thereof,
and recovering the resultant compound of formula Ib in free form or as appropriate in salt form.

8. A pharmaceutical composition comprising a compound according to any one of claims 1 to 5 in free form or as appropriate in pharmaceutically acceptable salt form together with at least one pharmaceutically acceptable carrier or diluent.

9. A compound according to any one of claims 1 to 5 in free form or as appropriate in pharmaceutically acceptable salt form for use as a pharmaceutical.

10. A compound according to any one of claims 1 to 5 in free form or as appropriate in pharmaceutically acceptable salt form for use in the prevention or treatment of conditions requiring immunomodulation, e.g. the modulation of unspecific antimicrobial resistance, or as an antiviral or anti-tumor agent.

Claims for the following Contracting States: ES, GR

1. A process for the preparation of a compound of formula I

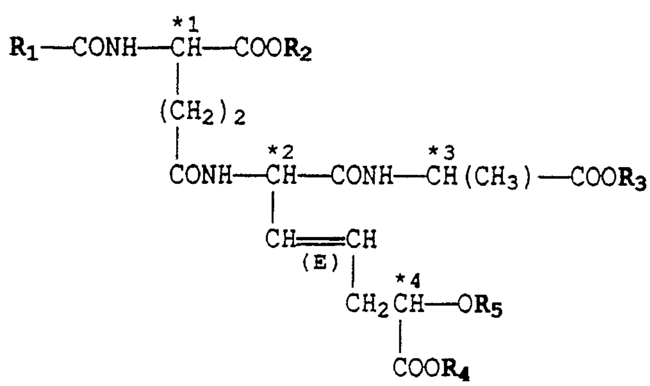

I

wherein
$R_1$ is alkyl or trifluoromethyl,
$R_2$, $R_3$ and $R_4$ independently are hydrogen or a carboxy-protecting group and
$R_5$ is hydrogen or alanyl optionally protected at the amino moiety thereof, with the proviso that when $R_2$, $R_3$ and $R_4$ simultaneously are hydrogen, then $R_5$ is hydrogen,
in free form or as appropriate in salt form,
comprising
a) for the preparation of a compound of formula Ia

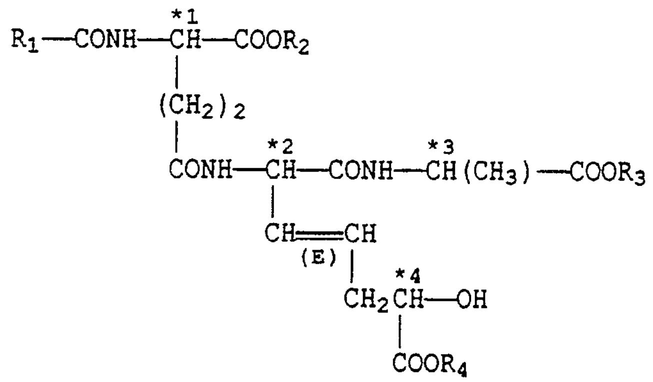

Ia

wherein $R_1$ to $R_4$ are as defined above,
reacting a compound of formula II

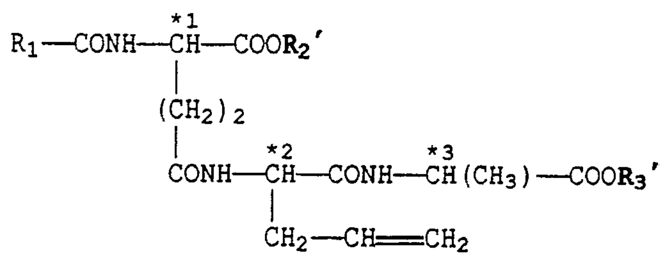

II

wherein
$R_1$ is as defined above and
$R_2'$ and $R_3'$ independently are a carboxy-protecting group,
with a compound of formula III
OHC - COO$R_4'$ III
wherein $R_4'$ is a carboxy-protecting group,
and where appropriate splitting off in one or more steps the carboxy-protecting groups from the resultant compound of formula Ia′

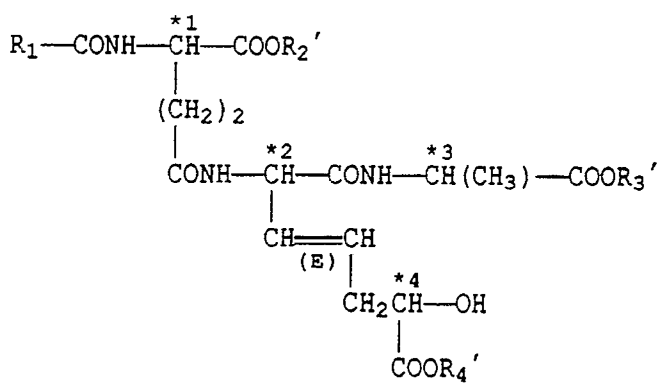

Ia′

wherein
$R_1$, $R_2'$, $R_3'$ and $R_4'$ are as defined above; or
b) for the preparation of a compound of formula Ib

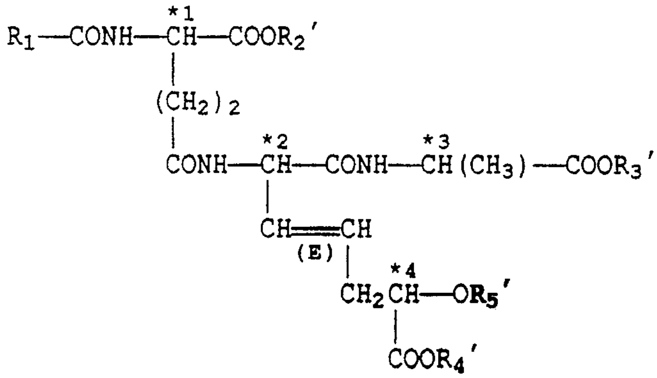

Ib

wherein

$R_1$, $R_2'$, $R_3'$ and $R_4'$ are as defined above and

$R_5'$ is alanyl optionally protected at the amino moiety thereof,

reacting a compound of formula Ia′ as defined above

with a compound of formula IV

$$HOOC - \overset{*5}{C}H(CH_3) - NHR_6 \qquad IV$$

wherein $R_6$ is an amino-protecting group,

and where appropriate splitting off the amino-protecting group from the resultant compound of formula Ib′

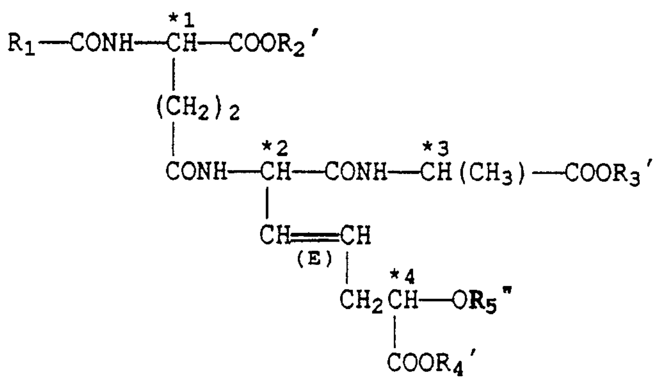

Ib′

wherein

$R_1$, $R_2'$, $R_3'$, and $R_4'$, are as defined above and

$R_5''$ is alanyl protected at the amino moiety thereof,

and recovering the resultant compound of formula I in free form or as appropriate in salt form.

2. A process according to claim 1 for the preparation of the compound of formula I wherein $R_1$ to $R_5$ respectively are Hex, Bz, Me, Bu and alanyl and the configuration at carbon atoms *1 to *5 respectively is either R, S, R, Y and S

or R, S, R, X and S,

in free form or as appropriate in salt form.

3. A process for the preparation of a pharmaceutical composition comprising mixing a compound of formula I as defined in claim 1 or 2 in free form or as appropriate in pharmaceutically acceptable salt form together with at least one pharmaceutically acceptable carrier or diluent.

European Patent Office

# EUROPEAN SEARCH REPORT

Application Number

**EP 90 11 7694**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | EP-A-0 231 087 (PFIZER)<br>* Page 2, line 1 - page 4, line 37 *<br>- - - | 1-10 | C 07 K 5/02<br>A 61 K 37/02 |
| A | JOURNAL OF ANTIBIOTICS, vol. XXXVI, no. 8, 1983, pages 1045-1050, Tokyo, JP; MINE et al.: "Immunoactive peptides FK-156 and FK-565. I. Enhancement of host resistance to microbial infection in mice"<br>* Page 1045, abstract *<br>- - - - - | 1-10 | |
| | | | **TECHNICAL FIELDS SEARCHED (Int. Cl.5)** |
| | | | C 07 K<br>A 61 K |

The present search report has been drawn up for all claims

| Place of search | Date of completion of search | Examiner |
|---|---|---|
| The Hague | 05 November 90 | KORSNER S.E. |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document